# EUROPEAN PATENT APPLICATION

(11) **EP 2 615 456 A1**
(43) Date of publication of application: **17.07.2013**
(21) Application number: 12185412.9
(22) Date of filing: 14.08.2009
(51) Int. Cl.: G01N 33/53, G01N 33/68

(54) **Method for identifying compounds which inhibit binding of anthrax protein to LRP5/6 receptors**

(30) Priority: 15.08.2008 US 228757
(62) Divisional of application: 09807359.6
(71) Applicant: ENZO BIOCHEM, INC., New York, NY 10022 (US)
(72) Inventor: Liu, Dakai, South Setauket, NY 11720 (US); Jin, Richard, Pennington, New Jersey 08534 (US); Li, Xiaofeng, Farmington, Connecticut 06032 (US); Zhang, Yazhou, South Setauket, NY 11720 (US); Cheng, Wei, Seaford, NY 11783 (US); Bhattacharyya, Riddhi, West Babylon, NY 11704 (US)
(74) Representative: Vossius & Partner

(57) **Abstract**

The present invention identifies compounds that disrupt the interaction between anthrax proteins and LRP5/6 receptors, resulting in a reduction in anthrax toxicity. The compounds act to disrupt the intracellular transport of toxin complexes into a target cell. The present invention also provides methods for testing the effect of compounds on Wnt activity, through the use *of in vitro* experiments involving cells that have in at least one gene mutation involved in the Wnt pathway.

## Description

### REFERENCE TO RELATED PATENT APPLICATIONS

This application claims the benefit of U.S. Provisional Patent Application No. 60/965,279, filed on August 17, 2007, entitled "Compositions and Methods for Bone Formation, Bone Remodeling and Toxin Protection" the contents of which are incorporated herein by reference.

This application is a Continuation-in-Part of Application Serial No. _______ filed on August 7, 2008, which is a Continuation-in-Part of Application Serial No. 11/598,916 filed November 14, 2006, which is a Continuation-in-Part of Application Serial No. 11/097,518 filed April 1, 2005, which is a Continuation-in-Part of Application Serial No. 11/084,668 filed March 18, 2005, which is Continuation-in-Part of Application Serial No. 10/849,067, filed May 19, 2004, the contents of all of which are incorporated herein by reference.

### FIELD OF THE INVENTION

The present invention relates to the field of therapeutic methods, compositions and uses thereof, in the treatment of bone fractures, bone disease, bone injury, bone abnormality, tumors, growths, viral infections, toxin poisoning, as well as for modulating pathophysiological processes including but not limited to glucose metabolism, lipid metabolism, triglyceride metabolism, adipogenesis, tumorigenesis, neurogenesis and bone-related activity. More particularly, the methods and compositions of the invention are directed to the use of mutagenesis to identify small molecules, drugs and/or pharmacological agents that affect the Wnt pathway by affecting normal complex formation among receptors (such as the anthrax receptor, for example), the LRP5 and LRP6 receptor, and related ligands.

### BACKGROUND OF THE INVENTION

The use of animal models or cell cultures with defects in one or more genes has become a standard technique for investigating the roles of such genes. The effects can be of an immediate nature where the lack of the gene product is studied directly or the effects can be of a more pleiotropic nature where the connection between the gene defect and the ensuing pathological processes is more distant. The latter is frequently observed in genetically determined metabolic diseases where the dysfunctional gene product has been identified, but the downstream exhibition of a number of seemingly disconnected symptoms are also seen. An example of this situation is Gaucher disease where the immediate effect is the lack of breakdown of glucosylcerebroside and more distantly related effects may include anemia, skeletal disorders, seizures, as well as lung and kidney impairment. An area of active investigational concern is why an imbalance in a particular process leads to a breakdown in other systems. In a disease like Gaucher, therapeutic studies can be carried out on the principle effect by finding ways to alter the cellular levels of glucosylcerebroside through the development of drugs that either decrease the rate of production or hasten the rate of its breakdown or excretion. The most successful treatment at the present time is the administration of glucosylcerebrosidase (sometimes referred to as enzyme therapy) to induce the breakdown of glucosylcerebroside. On the other hand, rather than treating the underlying cause (a buildup of glucosylecrebroside), therapeutic measures such as transfusions, bone marrow transplants and bone repair have previously been used before the introduction of enzyme therapy in order to ameliorate the various symptoms of the disease.

The existence of a large number of inherited diseases in human subjects and animal models offers a window into the mechanism and pathways of a variety of cellular and intracellular processes. However, such defects are only going to be observed with mutations that still allow viability of the subject. In most cases, this will be a defect that takes place in a heterozygous context where one chromosomal copy is defective but the homologous copy retains functionality, thereby allowing the inheritance of the disease condition. Under these circumstances, there is a reduction rather than elimination of a particular cellular process. The dispensability or indispensability of a gene function can often be seen in malting experiments with heterozygous animals with such mutations. Production of only wildtype or heterozygous offspring is an indication of the necessity of the gene function, whereas the production of homozygous viable offspring indicates at least some degree of dispensability. However, even in this latter case, viability may be a relative term where lifespans of homozygous offspring may average anywhere between a normal lifespan and mortality soon after birth. The effects of both heterozygous and homozygous conditions may also vary in terms of their presentation of genetic effects where there may be obvious physical parameters or there may be more subtle effects that are only seen after carrying out suitable assays.

Many mutations are the results of spontaneous processes where a particular trait is identified and later traced back to a defect in a specific gene. Homology studies have revealed that essentially the same gene may be present in widely different animals such as polyps, worms, insects, frogs and mammals. Although they may be involved in basically similar processes (development, for example), they are used for different purposes and mutations give rise to very different effects. An example of this can be seen with a variety of mutations in genes involved in the Wnt signaling pathway. For instance, Wnt itself was initially isolated and characterized as an oncogene (Nusse and Vannus 1982, Cell 31; 99-109). Genetic studies in Drosophila later identified a Wnt homologue where the defect was termed "wg" for "wingless" as a phenotype (Couso et al., 1995 Development 120; 621-636) and numerous studies have shown the role of varied members of this family of genes in development. In another example the Dkk family was named after an initial mutation in Xenopus (Glinka et al., 1998 Nature 391; 357-362) where overexpression generated a very large head size (Dkk was an abbreviation for "dickkopf", i.e. "fathead" in German). A role for this gene in mammals has also been seen where mice lacking Dkk1 show a lethal defect in head development during embryogenesis as well as polydactylism (Mukhopadhyay et al. 2001 Dev Cell 1; 423-434), yet on the other hand Dkk2 (-/-) mice are viable, fertile and look mostly normal (Li et a., 2005 Nature Genetics 37; 945-952, Mukhopadhyay et al., 2006 Development 133; 2149-2154). As will be discussed in more detail later, even genes as similar as LRP5 and LRP6 (which are frequently referred to in the literature as LRP5 and LRP6) show profoundly different effects when their gene activity is silenced where LRP5 (-/-) mice appear mostly normal and yet LRP6 (-/-) offspring die at birth.

The existence of mutations in the natural gene pool of humans and animal models has provided a large amount of information, but once genes and their functions have been identified it is possible to carry out a more direct approach of purposefully developing mutants in selected gene targets. For instance, animal laboratory strains have been created that are called "knockdowns" where genetic engineering has allowed the introduction of a cassette that synthesizes RNAi, thereby selectively decreasing the expression of a target gene. These models would be similar to animals or subjects with heterozygous (+/-) conditions, since there is a reduction rather than elimination of gene expression. For a given cassette, the level of repression may be variable since it will depend upon a number of factors, including whether there is one or more integration sites and the nature of the genomic environment. However, once a particular cell line has been isolated or an animal strain has been developed, the repression effect is a stable characteristic of that line or strain that allows observation of the effects induced by variations in levels of the gene product.

In some cases it is possible to create more stringent mutants called "knockouts" that are completely lacking in a particular gene function. These are also a product of genetic engineering where a gene target gene is disrupted by recombination with a nucleic acid construct that has a selective marker flanked by sequences homologous to the target gene. There can be either a deletion event where the marker replaces part of the target gene or the marker can be part of an insert. In either case the construct is designed such that a recombination event leads to a loss of function of the target gene. After characterization of the appropriate disruption event, these engineered cells are manipulated further and become part of a germ line transmission. The initial offspring is heterozygous (+/-) and studies can be carried out similar to those with spontaneous mutations. However, by inbreeding the heterozygous (+/-) animals, homozygous (-/-) offspring can be obtained. When the homozygous condition is a lethal event, a heterozygous (+/-) strain may be maintained and studies carried out on homozygous (-/-) offspring that proceed up to a particular stage of development. These studies have led to the understanding of critical stages where a gene product was absolutely required for further embryonic development. In contrast, the complete loss of function of a gene target can be less disruptive in some cases and viable offspring are produced with a homozygous (-/-) knockout condition that can be used to maintain a stable strain. The presence of the homozygous (-/-) condition may be revealed by a physically visible phenotype or aberrations may only be detectable or otherwise measurable using suitable assays.

This entire elimination of a gene usually produces different effects than those seen in spontaneous mutations since these can frequently be a point mutation. As such, the mutated copy sometimes affects only a portion of the gene product and varying degrees of functionality may still be retained. This effect has been seen in numerous cases where mutations in the same gene will express very different phenotypes depending upon the site and nature of the mutations. In contrast, an artificial knockout condition allows expression from only the single normal copy in the (+/-) heterozygote and absolutely no activity in the (-/-) homozygote.

As mentioned above, the properties of a homozygous knockout mutant can be profoundly different even when the targets are similar. For example LRP6 (-/-) knockout mice have a major defect in embryogenesis and never come to term (Pinson et al., 2000 Nature 407; 535-538). However, LRP5 (-/-) mice have normal embryogenesis and grow up into what appear to be essentially normal adults. A closer examination of LRP5 (-/-) mice shows the presence of a number of phenotypic traits that include osteoporosis (Kato et al, 2002 J. Cell Biol. 157; 303-314), defective eye vascularization (Gong et al. 2001 Cell 107; 513-523) and a defect in glucose induced insulin secretion (Fujino et al. 2003 Proc. Nat Acad. Sci (USA) 100; 229-234). Effects of mutations may also be augmented by the inclusion of other mutations as well. For instance, even though it has already been noted that LRP6 (-/-) has a lethal defect in embryogenesis, the further inclusion of a heterozygous mutation in the LRP5 gene (+/-) results in a more marked defect and embryos die shortly after gastrulation (Kelly et al., Development 131; 2803-2815). In a similar fashion, when LRP5 (-/-) mice also have a homozygous defect in the apoE gene they exhibit artherosclerosis and hypercholesteremia (Magoori et al., 2003 E J Biol Chem 278; 11,331-11,336), effects that are absent with either homozygous condition alone. For a review of the phenotypes of mice with knockouts in various genes of the Wnt signaling pathway, see Van Amerongen and Berns, 2006 (Trends Genet. 12; 678-689).

The essential role of some proteins presents difficulties in their studies. For example, a gene that is required for early embryonic events curtails studies on what effect a lack of this gene product might have in later stages. A solution to this conundrum has been the development of conditional mutations where functionality can either be maintained or repressed as desired during the lifespan of a test animal. This may be accomplished by processes similar to that used for the conventional knockdown and knockout mouse gene replacement modules but rather than carrying a constitutive effect on expression, there can be additional control elements such that its expression can be selectively altered or eliminated or it can have short sequences that allow an inducible deletion event through the Cre recombinase system. For a review of this technique, see Bockamp et al., 2002 (Physiol Genomics 11; 115-132)

It is obvious from the foregoing discussion that the individual members of a family of genes can play a variety of specialized roles. These may be due to variations in the structures of the proteins or subtle differences in amino acids at critical points that participate in protein/protein interactions. In addition to normal cellular or developmental functions, the receptors involved in signal pathways may also be used by foreign entities. For instance, the chemokine receptor CCR5 (Raport et all. 1996, J Biol Chem 271; 17,161-17,166) has also been identified as a co-receptor for infection by HIV-1 (Deng et al. 1996 Nature 382; 661-666, Dragic et al., 1996 Nature 381; 667-673). In another example, it has been shown that the lethal effects of anthrax toxin are produced by an interaction of the anthrax proteins with cellular membrane receptors of the host. The toxic activity of anthrax is principally due to the actions of two anthrax proteins, Lethal Factor (LF) and Edema Factor (EF), that bind to the anthrax Protective Antigen (PA) protein to form what is termed Lethal Toxin (LT). After transport of the LT complex into a cell by means of the endocytosis pathway, a subsequent release of the LF and EF into the cytosol produces the cytopathic effects of anthrax (Abrami et al., 2003 J Cell Biol 160, 321-328). However, entry into the cell was found to require binding to a cellular receptor termed the Anthrax Toxin Receptor or ATR. This receptor was isolated and the sequence identified as a previously described receptor called Tumor Endothelial Marker 8 (TEM8) which, in view of its additional potential function, is now also termed ATR or ANTRX1 (Bradley et al., 2001 Nature 414; 225-229). A second host receptor was later isolated and identified that could also participate in the transport of LT into the cell (Scobie et al., 2003 Proc Nat Acad Sci (USA) 100; 5170-5174). Previously characterized as Capillary Morphogenesis Protein 2 (CMG2), this protein is now also referred to as ATR2 or ANTRX2. For functionality, it has also been noted that the PA protein requires a preliminary protease reaction by the cellular protein furin after binding to one of the anthrax receptors that results in the exposure of sites used to bind the LF/EF proteins (Molloy et al., 1992 J. Biol Chem 267; 16396-16402).

In addition to either ANTRX1 or ANTXR2, it has recently been discovered that the binding to the host cell LRP6 receptor is also important in the transport of a complex through the cellular membrane to produce the toxic effects of anthrax (Wei et al., 2006 Cell 124; 1141-1154). The role of the LRP6 receptor was initially discovered by transfection with an Expression Sequence Tag (EST) antisense library and cells were assayed for protection against killing mediated by PA. When a colony that resisted high levels of toxin was examined for the particular EST present in the transformant, a single integrated sequence was identified as an intron portion of the LRP6 gene. A monoclonal antibody that recognized an epitope present in both LRP6 and the closely related protein LRP5 was used for Western blot analysis and demonstrated a severely decreased level of LRP5 and LRP6 expression compared to the parent cells. A loss of LRP activity was verified by a Wnt dependent assay with a β-catenin regulated promoter. As a separate method of testing the connection between anthrax resistance and the alteration of LRP6, siRNA constructs were specifically designed to block LRP6 and transformants were shown to have an increased rate of survival. Direct tests were then undertaken that showed that the amount of dye labeled PA was reduced on both the surface and in the cytoplasm of cells that had either the antisense or siRNA constructs suggesting that both binding and internalization of PA was affected by the lack of an adequate amount of LRP6. A dominant negative mutant form of LRP6 was created that was fully functional for the extracellular domains but lacked most of the intracellular segment; protection could also be provided by this mutant protein although in this case binding to the truncated product was observed as being normal and only cellular entry was blocked. To exploit these effects, a polyclonal antibody was raised against a 20 amino acid sequence derived from the second domain of LRP6. Administration of this agent prevented cellular uptake of the anthrax complex thereby abolishing its lethal effects. In contrast, an antibody raised against the first repeat domain of LRP6 showed no effect at all, demonstrating a specificity for a particular site on LRP6 for binding to the anthrax complex. In addition, immunoprecipitation experiments showed binding between the LRP6 and the TEM8 or CMG2 receptors while no direct binding was observed between LRP6 and PA itself. However, interactions between the anthrax proteins and LRP6 are possible since it was observed that the binding affinity between CMG2 and LRP6 was increased by the addition of PA. The binding of PA may induce a conformational change in CMG2 that increases its affinity for LRP6 or alternatively, the binding affinity is increased through direct interaction of PA with LRP6 but only after it binds to CMG2. Due to the high level of structural homology between LRP5 and LRP6 in the region involved in the interaction of LRP6 with the cellular receptor, it is possible that LRP5 may substitute for LRP6 as a co-receptor for anthrax importation. In such a case, the ability of Wei et al. to induce anthrax resistance by disrupting only LRP6 may be a consequence of a lack of effective amounts of LRP5 in the particular cells they were using.

The interaction of the proteins involved in the lethality of anthrax toxin has been the subject of numerous studies. As described previously, the anthrax proteins form the Lethal Toxin or LT complex prior to intracellular importation. The LT complex is formed by a heptameric complex of PA with three molecules of LF and/or EF. Detailed studies on this complex have established the positions of the particular sites on the anthrax proteins that are involved in protein/protein interactions. Thus, the site on the PA component that interacts with LF and/or EF has been identified as Domain I (Petosa et al. 1997 Nature 385; 833-838). Mutational analysis was then able to identify particular amino acids in PA responsible for this binding (Chauhan and Bhatnagar, 2002, Infect Immunol 70; 4477-4484, Cunningham et al., 2002 Proc. Nat. Acad Sci (USA) 99; 7049-7083). For the corresponding interaction sites on LF and EF, it had been previously noted that two segments referred to as LF_{N} and EF_{N} respectively, are similar in terms of both sequence and structure (Pannifer et al., 2001 Nature 414; 229-233). As expected, these homologous segments are the domains that interact with PA (Elliot et al., 2000 Biochemistry 39; 6706-6713). Alanine scanning was then later used to identify a series of amino acids of EF involved in binding with PA (Lacy et al., 2002 J Biol Chem 277; 3006-3010).

The sites of the interactions between PA and cellular receptors have also been characterized for the former (PA), as well as the latter (TEM8 and CMG2). Determination of the crystal structure as well as biochemical data has led to the discovery that Domain 4 of the anthrax PA protein is involved in binding to the CMG2 and TEM8 cellular receptors (Petosa et al. 1997 Nature 385; 833-838). Alanine scanning of the surface of this Domain 4 portion (Rosovitz et al., 2003 J Biol Chem 278; 30,936-30,944) has revealed amino residues that are important in binding to the cellular receptors as well as a neutralizing antibody 14B7 that had previously been shown to block binding of PA to cells (Little et al., 1988 Infect Immun 56; 1807-1813). Further studies have shown that the interaction is more complex and that Domain 2 of the anthrax PA protein is also involved in interactions with the cellular CMG2 (and presumably TEM 8) receptor (Lacy et al. 2004 Proc Nat Acad Sci (USA) 13,147-13,151). As such, mutational analysis has also been extended to sites in Domain 2 of PA (Liu et al., 2006 Cell Microbiol).

The other side of the interaction (cellular receptors TEM8 and CMG2) concerning the particular portion of the cellular receptors that bind to the anthrax proteins is also known. In the reports on the identification of TEM8 and CMG2 as anthrax receptors, a sequence referred to as a Van Willebrand factor A (VWA) Domain was noted as being held in common by both of the receptors with an approximate 60% homology for this region. Since this type of sequence has been previously observed to be involved in protein/protein interactions, experiments were carried out that showed that soluble recombinant VWA domains from TEM 8 and CMG2 are able to bind to PA (Bradley et al., 2001 Nature 414 225-229, Scobie et al., supra). Mutagenesis analysis has also been carried out on CMG2 to identify important amino acid residues in this region (Liu et al., supra).

### SUMMARY OF THE INVENTION

The present invention discloses the use of compounds that disrupt the interaction between anthrax proteins and LRP5/6 receptors in order to reduce anthrax toxicity. The discovery that transport of anthrax toxins seems to involve the binding to Domain II of LRP6 allows the use of compounds that bind to this site in order to disrupt intracellular transport of toxin complexes into a target cell. Molecules that have been previously described as binding to one or more of the YWTD domains of LRP5 and LRP6 and blocking Wnt and/or Dkk activity may be used for this purpose. Alternatively, compounds that have been selected for YWTD binding but have not shown utility in affecting the Wnt pathway may still have the ability to block anthrax toxicity and may be specifically tested for this function.

In another embodiment of the present invention, a method for testing the effect of candidate compounds on Wnt activity is described where in vitro experiments are carried out in cells that are mutated in one or more of the genes involved in the Wnt pathway. This alteration in the genetic environment of the assay may simplify effects by reducing of the number of possible pathways taking place in these cells. This method may reveal effects that in an otherwise normal cell may be a net product of competing effects, therefore allowing an optimization of pharmaceutical agents for a desired process. The method also allows the testing of compounds that effect alternative pathways in order to design a multidrug method of treating a disease.

### BRIEF DESCRIPTION OF THE DRAWINGS

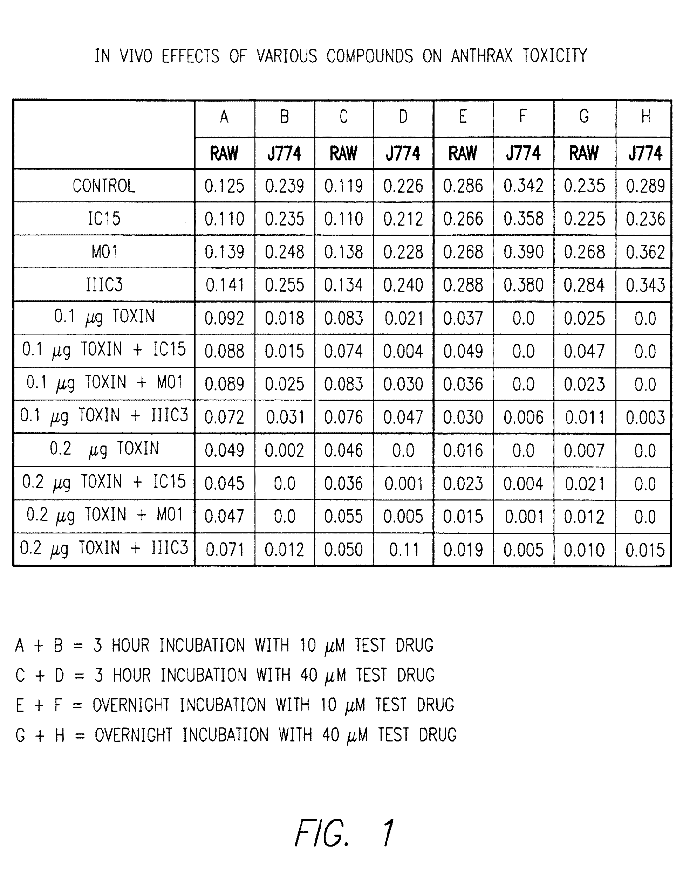

Figure 1 shows a Table with results obtained for anthrax toxicity by various compounds.

### DETAILED DESCRIPTION OF THE INVENTION

In previous art, mutations in subjects or animal models have been used for the purpose of elucidating pathways for various conditions and diseases. A novel aspect of the present invention is that mutant animals or cells can be used for testing the efficacy of potential pharmacological agents after carrying out a physical or virtual screening of a library. In a preferred embodiment of the present invention, a mutation is located in one or more genes of the Wnt canonical or non-canonical signaling pathway. In the course of carrying out a virtual screening, it is understood that the library itself can be a physical library (as exemplified by screening of the NCT library in U.S. Patent Application Serial No. 2005/0196349, herein incorporated by reference) or it can be a virtual library (as exemplified in Example 6 with compounds Enz-1 to Enz-72 of U.S. Patent Application Serial No. 11/598916, herein incorporated by reference). Any compound that can potentially bind to the protein target of interest and affect the interaction between the target protein and another protein may be selected as a member of the library. Examples of such compounds can include but not be limited to organic molecules, antibodies, peptides, and nucleic acids. The peptides can include, but not be limited to, a library of peptides of random nature, a permutational series of amino acids, fragments of antibodies to the protein of interest and fragments of a protein that interacts with the protein of interest. The nucleic acids can include but not be limited to aptamers and a library of protein binding sequences.

When the user has access to a physical library, the structure of each member may be used in a virtual screening process and candidates of interest may be subsequently tested. In contrast, there is no absolute necessity to have such molecules immediately in the possession of the user and if some data has already been collected on particular compounds, their structure may be used to design a virtual library with variations in the positions on core structures followed by a virtual screening process. In this variation, a wide variety of related compounds may be analyzed simultaneously and only the particular compounds that score highest after the virtual screening process need to be synthesized and tested in biological assays.

In the human genome, gene duplication events have led to the existence of a certain degree of redundancy such that multiple copies of similar proteins carry out similar functions. In some of these cases, there are more or less complete copies that are expressed from different genomic sites and in other cases there are families of proteins where there may be differences between otherwise identical copies that reflect evolutionary developments that have led to alterations of some properties, a process that is sometimes referred to as genetic drift. In some cases, this differentiation has led to specialization where particular functions are carried out only by certain members of such families. In other cases, there may be functional overlaps where either of two proteins of a given protein family can carry out a specific step. As a further complication, two unrelated proteins may also be carrying out a particular step in common due to convergent evolution. When two different proteins (related or unrelated) are able to carry out or initiate a common process, there may be difficulties in identifying pharmacological agents that can modulate this process. In such an instance, there may be masking by the presence and activity of a second protein when screening for the activities of a pharmaceutical agent specifically selected for potential inhibition of a target protein, i.e. even when the first protein is effectively blocked by a particular drug candidate, a lack of effective repression of the second protein can lead to little or no effect being seen in the assay system. Thus, a molecule that is highly selective for the first protein may be completely missed by the screening procedure. In the present invention, it is disclosed that animals and cells with mutations in the Wnt signaling system of either natural or artificial origin may provide a more effective means of selecting drug candidates. Whereas in previous art, a partial or complete loss of function of a particular gene was used for delineation of the role of a gene or as a model system for development of therapies that compensate for its loss, the present invention uses the loss of function in one gene to allow identification of pharmacological agents that affect a different gene. In a preferred embodiment of the present invention, these mutations are in proteins involved in the Wnt signaling pathway. Thus, to give a non-limiting example, when a drug is being tested for an ability to inhibit the activity of LRP6, the present invention discloses the utility of carrying out a biological assay procedure in an LRP5 (-/-) environment.

In a system where either of two proteins is capable of transmitting a signal, this process may also be of a reciprocal or sequential nature. For instance, once an effective drug has been identified in a cell or animal where the presence of a mutation has made signal generation dependent upon only the first protein due to partial or complete elimination of the activity of the second protein, the same procedure can then be carried out in a second stage with cells or strains that are defective in the first protein and screening a library for compounds that have the ability to block the second protein. Thus, in a system where a particular step can be carried out in parallel by more than one protein, a potential benefit of the present invention can be treatment by a combination of therapeutic agents that are optimized for each target. On the other hand, once a compound is identified that is effective with the first target, a series of modifications can be carried out with this compound to identify a pharmacological agent that is effective on the second target as well as the first, thereby taking on a multi-targeting role.

There are also situations where a pharmaceutical agent can inhibit the actions of a target protein in a cell, but the presence of a second protein, unrelated to the first, may compensate for this effect and nullify any results. In this case, the same strategy outlined above may be used where a mutant lacking the second protein can allow a more fruitful investigation of screening for agents that affect the target protein. As also described above, a second search can then be carried out later for a small molecule that can separately affect the second protein such that a desirable effect can be obtained by a combination of agents that affect the first and second protein targets individually.

### Interactions with Anthrax

It has already been established that the LRP5 and LRP6 receptors are involved in a number of different protein/protein interactions for carrying out signal transduction events. As described above, it has also been found that a binding event to the LRP6 receptor can lead to transportation of a complex through the cellular membrane to produce the toxic effects of anthrax (Wei et al., 2006). By directing antibodies to two different sites on the LRP6 protein, blockage of one site was shown to be effective in reducing the effects of anthrax toxicity while blocking of the second site seemed to offer no protective benefits. The ineffective site was located on a region corresponding to the third repeat of Domain I (amino acids 204-213) while the resistance inducing site was located in the third repeat of Domain II (amino acids 515-534) implying that binding of the anthrax complex to Domain II may be an important factor in the toxicity of anthrax. As such, the same methods that have been previously described for Identification of molecules that modulate interactions of LRP5 and LRP6 receptor with other proteins (U.S. Patent Application No. 2005/0196349) may also be used to identify a molecule that can interfere with anthrax induced toxicity.

The oligopeptides used by Wei et al. for raising the polyclonal antibodies against the YWTD repeat Domain II region were derived from LRP6. It is unknown whether there was activity against the corresponding sequence in LRP5 since the homologous sequence in LRP5 only matched 13 out of the 20 amino acids. However, it is possible that the presence of conserved amino acids in Domain II (as well as the similarity in structure) allowed blockage of the corresponding LRP5 site by the polyclonal antibody. In contrast, there may not have been expression of LRP5 in that particular cell line and as such, it did not have to be blocked. Since structures and functions are so similar, it is probable that when LRP5 is expressed, it may also act as a co-factor for anthrax toxicity. It is therefore an object of the present invention to identify molecules that bind to LRP5 as well as to LRP6. As described above, compounds may be identified that bind to both LRP5 and LRP6, or there may be compounds that bind to LRP65 and LRP6 separately.

There are two approaches that may be used in the present invention. The first approach is a site-selective method where the binding site on LRP5 and LRP6 that has been identified as both a binding site for a native protein and for the anthrax protein complex is screened using information gained from the native protein. In this approach it is assumed that a molecule which is able to block the action of the normal protein may also offer protection against the anthrax protein complex that binds to the same site. The benefit to this is that it takes advantage of screenings and findings from investigations of small molecules that modulate interactions between the YWTD repeat domain of LRP and the native protein.

An example of this method is using the candidates that have been identified as modulating the interaction between LRP5 and Dkk and testing for an additional property of being able to offer protection against anthrax toxicity. Molecules that have been selected on the basis of inhibiting the interaction of Dkk with LRP6 may also be used for this purpose.

An alternative approach is to carry out a more focused ligand-selective method where the same methodology that has been used for identifying the site on LRP5 for Dkk interaction is used to more specifically identify the site used for anthrax. As described in U.S. Patent Application No. 2005/0196349, key amino acids in LRP5 involved in the binding of Dkk to YWTD Domain II were identified by alanine scanning prior to carrying out a virtual screening that used the amino acid locations as interaction sites. Due to the similarity between Domains II and III, molecules that have been selected for binding to one site may be able to bind to the other site and similarly, compounds selected for their ability to bind to LRP5 may also bind to LRP6. However, a more selective approach for the present invention would consist of carrying out a mutational program to identify the sites of amino acids in Domain II and Domain III of both LRP5 and LRP6 that may be critical for the translocation of anthrax into a cell. It may be of further benefit to combine this aspect of the present invention with other previously disclosed methods involving the use of mutants. In this combined approach, mutants are used to develop a cell line where both LRP5 and LRP6 are eliminated, and anthrax susceptibility is determined by transformation with appropriate LRP5 or LRP6 constructs. In addition, the structure of compounds that show inhibitory activity may be used to carry out further rounds of virtual screening as previously disclosed in U.S. Patent Application No. 2005/0196349.

Wei et al. used a peptide with amino acids 1314-1613 of LRP6 to generate a third polyclonal antibody that also displayed effectiveness in protection against anthrax toxicity. These amino acids comprised a small portion of the extracellular domain (1314-1370) as well as a portion of the intracellular domain (1394-1613). Presumably, it is the extracellular portion that was recognized by the antibodies in the experiments carried out by Wei et al., and this result represents either an additional binding site of the anthrax complex, an alteration in secondary structure that interferes with binding to a distant binding site, or an interference with the endocytosis process. Regardless of the mechanism, these results imply that this region may also serve as a target for the identification of a small molecule that could interfere with anthrax toxicity.

In previous art, blocking the lethal effects of anthrax infection/exposure has been the subject of a tremendous amount of research. Although the bacillus itself is susceptible to a number of different antibiotics, the effects of the toxin can create lethality even after the disease organism itself has been eliminated. That is, after a certain stage of infection, antibiotics have no effect when the anthrax toxins are present in sufficient amounts. As such, recent efforts have been more directed towards blocking the effects of the toxin itself rather than destroying the organism that carries it. This has been a brute force screening approach for testing the effects of a library of compounds on cells and animals. Assays were carried out that either looked at particular steps of the anthrax toxin pathway or simply assessed overall lethality.

An undirected approach has been to take previously described drugs that effect multiple targets to reduce anthrax toxicity and test them for an additional ability to be used in anthrax intervention. For instance, the anti-cancer drug cisplatin is known to affect a wide range of processes during treatment of various disorders and it was shown that cisplatin could block anthrax toxicity when it was used to treat the PA prior to its administration (Moayeri et al., 2006 Antimicrob Agents and Chemotherapy 50; 2658-2665). In vivo experiments also displayed an effect when the drug was co-administered with lethal (anthrax) toxin (LT). However, practical results for the use of this drug are lacking. Co-administration was a critical factor. The administration of cisplatin two hours before or two hours after administration of the lethal toxin eliminated this protection.

Since one of the steps of anthrax toxicity is the protease action on critical cellular targets by the anthrax LF protein, this activity has been the subject of both random screening and rational drug design efforts where the structure of the LF protein has been used to identify appropriate inhibitors. Examples of the former have included the testing of 10,000 "drug-like" molecules using a non-selective physical screening approach for the identification of LF inhibitors (Schepetkin et al., 2006 J Med Chem 49; 5232-5244). A more selective variation of this approach has been to take into consideration the presence of anionic rich regions on the LF protein, and physically test for inhibition by a small library of cationic compounds (Goldman e al., 2006 BMC Pharmacology 6:8-15). An example of a rational drug design approach has been the combination of crystallography, molecular docking (virtual screening) and data mining to identify compounds that could bind to LF and thereby inhibit its protease activity (Panchal et al., 2004 Nat Struct Mol Biol 11; 67-72). Other examples of the drug design approach have included the use of the crystallographic predicted structure of LF to select a primary "scaffold" from a group of three hundred "scaffolds" that represent various drug families (Forino et al., 2005 Proc Nat Acad Sci (USA) 102; 9499-9504), thereby limiting the amount of searching required. Once a primary structure was selected, a search was made for related compounds that were commercially available. In vitro testing followed to determine the parts of the core compound that needed to be retained for the maintenance of inhibitory activity. Subsequently, structure activity relationship (SAR) analysis was carried out to design novel compounds that could be tested further. (see Johnson et al., 2006 J. Med Chem 12; 27-30). A mixed approach has incorporated the use of a random peptide library to identify the optimal peptide substrate, followed by the design of peptide analogs that could act as inhibitors (Turk et al.,. 2004 Nat Struct Mol Biol 11; 60-66). This work was continued by carrying out crystallography studies of the inhibitor bound to LF in order to refine designs for more drug candidates. In addition, as previously described, that drugs that have proved to be useful in other contexts (cisplatin) have also been retested for their application to anthrax. Others have examined the ability of some previously developed metalloprotease inhibitors to inhibit anthrax toxicity due to blockage of the activity of LF on cytosolic targets (Kocer et al. 2005 Infection and Immunity 73; 7548-7557).

The application of compounds directed to intracellular targets is problematic because there must be active or passive transport of the compound into the cell. As such, there may be a number compounds that may affect anthrax toxic activity that are ineffective in cellular assays because of an inability to enter the cell. Since anthrax toxin action is initiated by events taking place on the cell surface, compounds that affect events taking place in this extracellular environment can avoid such problems and provide a greater realm of potential pharmacological agents. As such, rather than aiming directly at LF enzymatic activity, a search has also been carried out for compounds that would bind to the PA protein such that the entry of LF into the cell would be blocked (Karginov et al., 2005 Proc Nat Acad Sci USA 102; 15,075-15,080). As previously mentioned, cisplatin was used for the inhibition of anthrax toxicity. Although it was partially chosen for working in the intracellular environment as a known protease inhibitor, it seems that its effectiveness in blocking anthrax in vivo may be taking place by blocking translocation of LF into the cytosol (Moayeri et al., 2006).

Various events occur prior to the translocation of the anthrax toxin complex into the cell. Consequently, these pre-translocation events are also potential targets for pharmacological intervention. A prerequisite for translocation is a protease cleavage of the PA protein by the endogenous protease furin. In one report on the use of furin as a target for a drug such as endogenous protease inhibitor (inter-alpha-inhibitor protein) was found to increase the survival of treated animals (Opal et al., 2005 Infect Immun73; 5101-5105). Other furin inhibitors have also been isolated that are either modified proteins or functionalized small peptides (Komiyama et al., 2005 Antimicrob Agents Chemotherapy 49; 3875-3882). The reagents described in this study showed protection against toxin lethality for at least 5 hours, but after 8 hours the course of lethality resumed, i.e. these agents did not seem to prevent toxin lethality *per se* but only delayed it. This resurgence of lethality could partially be prevented by the co-administration of a second reagent, chloroquine, at the same time as the furin inhibitor. In addition to incomplete protection, there could also be an immune reaction to these peptides. The use of peptides and/or proteins may also have problems with stability where specific storage requirements are needed. This could be problematic when application of these reagents may be needed immediately in bio-warfare conditions where a pill or desiccated powder may be more useful.

In another example on the selection of extracellular targets, advantage has been taken of the knowledge that protein/protein interactions are an important element in the lethality of anthrax toxin. For instance, a random peptide library was used in a phage display system to screen 7 or 12 amino acid peptides that would bind to Domain I of ANTRX1 and ANTRX 2 (Basha et al., 2006 Proc Nat Acad Sci (USA) 103; 13,509-13,513). At least one peptide selected on the basis of binding to ANTXR1 was later shown to be able to inhibit anthrax toxicity in a cell line (RAW 264.7) that expresses ANTRX2. These studies showed that the simultaneous administration of the peptide as well as the lethal toxin blocked the lethality of the anthrax toxin in vivo. However, the use of peptides also entails problems cited previously that might abrogate their utility.

As described above, the discovery that LRP6 was also a co-receptor for the translocation of anthrax toxin into a cell was partially based upon the use of antibody to specific regions of LRP6 (Wei et al., 2006). This observation has been used as the basis of a therapeutic mode, where Cohen and Wei have disclosed the use of monoclonal antibodies against LRP6 as reagents that could inhibit anthrax toxicity in vivo (U.S. Patent Application No. 20060257892 filed Feb 16, 2006). This is similar to certain previously described methods where a specific target is chosen and then a screening of a random library of potential inhibitors is carried out where candidates are evaluated on the basis of their ability to bind to LRP6. Although the main concern of this application is the use of antibodies and variations thereof as reagents, there is also the potential use of small molecules. However, this method is carried out in the same way previously described for searching for antibodies and the discussion on screening is concerned solely with biological assays. Although screening of a random library by a biological assay is the only way to carry out a search for antibodies, this does not hold true for small molecules where more sophisticated ways are available. There is no suggestion or appreciation in the Cohen and Wei application that a much more efficient system is the method described in the present invention that uses the structure of LRP6 (and possibly LRP5) to carry out a virtual screening of a library of compounds prior to carrying out a series of biological assays.

Effective molecules that are discovered by using the materials and methods of the present invention may be used in conjunction with pharmacological agents that have been selected for intervention in other steps in the process leading to anthrax toxicity. It has previously been shown that reagent combinations have provided more effective protection against anthrax toxicity compared to being used alone (Komiyama et al., 2005). It would be expected that the use of a new target by means of the present invention should allow these compounds to enjoy cumulative or even synergistic effects when used with other anti-anthrax reagents.

The compounds of the present invention and the compounds identified by the methods described in U.S. Patent Application 2005/0196349 and related applications may be used in conjunction with one or more other drugs in the treatment, prevention, control, amelioration, or reduction of risk of diseases or conditions for which the compounds of the present invention have utility, where the combination of the drugs together are safer or more effective than either drug alone. Examples of combinations of these compounds with other drugs in either unit dose or kit form include combinations with: a) antiresorptive reagents, such as Bisphosphonates (for example, Alendronate sodium, sold under the brand name Fossamax® by Merck); b) anabolic reagents, such as Parathyroid hormones (e.g., Teriparatide, a recombinant form of parathyroid hormones sold under the brand name Forteo® by Eli Lilly); c) bone regeneration material, such as beta-tricalcium phosphate (i.e. beta-TCP, sold under the brand name Cerasorb® by Curasan AG); and 4) other drugs that affect receptors or enzymes that either increase the efficacy, safety, convenience, or reduce unwanted side effects or toxicity of the compounds of the present invention or the compounds in related applications. The foregoing list is illustrative only and not intended to be limiting in any way. An advantage of this approach includes the possibility of synergistic effects where the products of two different modalities may be more beneficial than a single medicine. Also where the same level of relief is achieved by different medicines, this treatment may be carried out by using lower dosages of two or more medicines resulting in a diminishment in potential side effects that would be seen with a higher dose of any single medicine.

When carrying out the methods of the present invention, treatments may be chosen from a variety of administration methods comprising but not limited to oral, nasal, inhalation, intravenous, intraperitoneal, intramuscular, parenteral, transdermal, sublingual, topical, rectal or subcutaneous means. When carrying out a combination procedure, the treatments may share the same administration or treatment method or they may utilize different methods. The pharmacological agents identified by the present invention may also be administered with other agents as well that can include but not be limited to excipients, drug release-polymers, carriers, and enhancers.

The molecules or compounds identified by the methods of the present invention may be used to create compositions and/or pharmaceutical compositions that may be administered to subjects or patients (in therapeutically effective amounts) to treat disorders, diseases or conditions that are affected by modulating the activity of any member of the Wnt signaling pathway.

The compounds or molecules of the present invention may have asymmetric centers, chiral axes, and chiral planes (as described in: E.L. Eliel and S. H. Wilen, Stereochemistry of Carbon Compounds, John Wiley & Sons, New York, 1994, pages 1119-1190), and occur as racemates, racemic mixtures, and as individual diastereomers, with all possible isomers and mixtures thereof, including optical isomers, all such stereoisomers being included in the present invention.

The compounds or molecules of the invention, and derivatives, fragments, analogs, homologs pharmaceutically acceptable salts or hydrate thereof, can be incorporated into pharmaceutical compositions suitable for administration, together with a pharmaceutically acceptable carrier or excipient. Such compositions typically comprise a therapeutically effective amount of any of the compounds above, and a pharmaceutically acceptable carrier.

The compounds or molecules of this invention may be administered to mammals, preferably humans, either alone or, preferably, in combination with pharmaceutically acceptable carriers, excipients or diluents, in a pharmaceutical composition, according to standard pharmaceutical practice. The compounds or molecules can be administered orally or parenterally, including the intravenous, intramuscular, intraperitoneal, subcutaneous, rectal and topical routes of administration.

The subject or patient to whom the compounds of the present invention is administered is generally a human being, male or female, but may also encompass other mammals, such as dogs, cats, mice, rats, cattle, horses, sheep, rabbits, monkeys, chimpanzees or other apes or primates.

The terms "administration of" or "administering a" compound should be understood to mean providing a compound of the invention to the individual in need of treatment in a form that can be introduced into that individual's body in a therapeutically useful form and therapeutically useful amount, including, but not limited to: oral dosage forms, such as tablets, capsules, syrups, suspensions, and the like; injectable dosage forms, such as IV, IM, or IP, and the like; transdermal dosage forms, including creams, jellies, powders, or patches; buccal dosage forms; inhalation powders, sprays, suspensions, and the like; and rectal suppositories.

The terms "therapeutically effective amount" means the amount of the subject compound that will elicit the biological or medical response of a tissue, system, animal or human that is being sought by the researcher, veterinarian, medical doctor or other clinician. As used herein, the term "treatment" refers to both to the treatment and to the prevention or prophylactic therapy of the mentioned conditions, particularly in a patient who is predisposed to such disease or disorder.

The term "treating" in its various grammatical forms in relation to the present invention refers to preventing, (i.e., chemoprevention), curing, reversing, attenuating, alleviating, minimizing, suppressing or halting the deleterious effects of a disease state, disease progression, disease causative agent (e.g., bacteria or viruses) or other abnormal condition. For example, treatment may involve alleviating a symptom (i.e., not necessary all symptoms) of a disease or attenuating the progression of a disease. Because some of the inventive methods involve the physical removal of the etiological agent, the artisan will recognize that they are equally effective in situations where the inventive compound is administered prior to, or simultaneous with, exposure to the etiological agent (prophylactic treatment) and situations where the inventive compounds are administered after (even well after) exposure to the etiological agent.

### EXAMPLES

Examples provided are intended to assist in a further understanding of the invention. Particular materials employed, species and conditions are intended to be further illustrative of the invention and not limited of the reasonable scope thereof.

### Example 1

### Protection against anthrax toxicity

### A. Test Compounds

In US Patent Application Serial No. 2005/0196349, a virtual screening was disclosed that identified compounds that could interact with Domain III of LRP5; potentially interesting compounds were then tested in a biological assay for an ability to modulate Wnt activity. Two of the compounds that were a result of this process were IC15 and IIIC3. As described in US Patent Application No. 11/598,916, compound IIIC3 was used to design a series of related compounds by varying functional groups on a core structure. One of the products that gave positive results by both virtual screening scores and biological assays was the compound Enzo MO1. As described above, compounds that have been selected to bind to Domain III of LRP5 might be able to give protection against anthrax toxicity.

### B. Preparation of Test Compound Stocks

Compounds IC15, IIIC3 and EnzoM01 were prepared as 312.5 µM concentrated stock solutions and diluted into culture media as 4x stocks such that a final concentration of 10 and 40 µM would be present during the assay.

### C. Preparation of Anthrax Toxin Stocks

PA and LF proteins (0.1 mg/vial, List Biological Labs Inc.. Campbell, CA) were reconstituted with 100 µl of H₂O to give a final concentration of 1 mg/ml. These were aliquoted into separate 10µl samples that were maintained at 20-80°C until required. Working solutions of PA and LF were made by diluting 1 mg/ml of each toxin stock into media to give a final concentration of 10 µg/ml. PA and LF were diluted and mixed together just prior to use to give a 4x toxin mix that resulted in either a 0.1 µg/ml or 0.2 µg/ml final concentration during the assay.

### D. Biological Assay

Two murine macrophage-like cell lines, J774A.1 (ATCC TIB-67) and RAW264.7 (ATCC TIB-71), were used to examine the effects of the selected compounds on anthrax toxicity. Cells were grown in DMEM medium supplemented with 4mM GlutaMax-1, 4.5 g/L glucose, 1.5 g/L sodium bicarbonate, 10% FBS, 1% Penicillin/Streptomycin and buffered with HEPES followed by seeding 5 x 10³ cells/well into a 96 well plate. After overnight growth, 50 µl of a test compound diluted into media was added to the 100 µl of medium already present in each well and incubated for 30 minutes. At this point, 50 µl of 4x toxin was added to give a final volume of 200 µl. At various time points (3 hours or overnight), medium was collected and cells washed once with 180 µl of 1x PBS, followed by addition of 20 µl of MTS reagent (Promega, Madison WI) mixed with 100 µl of RPMI 1640 medium (phenol red free) supplemented with 1% FBS to measure vitality. Incubation was then carried out for 2-4 hours followed by absorbance readings at 490 nm and 630 nm. The results of the 490 nm results are tabulated in Figure 1. Each sample was carried out in triplicate and the numbers shown in Figure 1 represent an average of the three. Background level subtractions were 0.073 for the 3 hour incubation samples and 0.083 for the overnight samples, both backgrounds being established from control samples without cells.

### E. Discussion of Results

It can be seen that under the conditions used, the J774 cell line is more sensitive to anthrax toxicity than the RAW264 cells. For the J774 cells, some effects of protection were provided by M01 and IIIC3 during the three hour incubation period which was essentially lost by extending the incubation to overnight exposure or increasing the toxin from 0.1 µg to 0.2 µg. For the RAW cells, little or no resistance was seen with any of these compounds after the three hour incubation, whereas in the overnight exposure, IC15 seemed to offer limited protection in the presence of either 0.1 µg or 0.2 µg of toxin. Such differential effects may be due to the nature of the sensitivity of J774 compared to RAW264 or it may be related to different expression patterns of anthrax receptors for these cell lines.

Furthermore, the present invention relates to the following items:
1. A method for identifying a molecule or compound that modulates the activity of a member of the Wnt signaling pathway comprising the steps of:
   (i) determining the structure of a target protein that is part of the Wnt signaling pathway;
   (ii) carrying out a mutational analysis of said target protein wherein said analysis defines a binding cavity;
   (iii) carrying out a virtual screening of a library of compounds to assess the binding affinity of the compounds to said cavity;
   (iv) selecting compounds based on their binding affinity; and
   (v) carrying out a biological assay of selected compounds resulting in the modulation of Wnt activity in a cell or animal.
2. A method for identifying a molecule or compound that modulates the activity of a member of the Wnt signaling pathway comprising the steps of:
   (i) determining the structure of a first protein that is part of the Wnt signaling pathway;
   (ii) carrying out a virtual screening of a library of compounds to assess the binding affinity of the compounds to a binding cavity on said first protein which interacts with a second protein involved in the Wnt pathway;
   (iii) selecting compounds based on their binding affinity;
   (iv) providing a cell with a mutated gene resulting in the reduction or elimination of the expression of a third protein involved in the Wnt signaling pathway; and
   (v) testing said selected compounds in said mutated cell with a Wnt-dependent biological assay that measures a change in Wnt activity caused by the presence of said selected compounds.
3. A method for identifying a molecule or a compound that modulates the activity of a member of the Wnt signaling pathway comprising the steps of:
   (i) determining the structure of a first protein that is part of the Wnt signaling pathway;
   (ii) carrying out a virtual screening of a library of compounds to assess the binding affinity of the compounds to a binding cavity on said first protein which interacts with a second protein involved in the Wnt pathway;
   (iii) selecting compounds based on their binding affinity;
   (iv) providing a cell with a mutated gene resulting in the reduction or elimination of the expression of a third protein in the Wnt signaling pathway which binds with said first protein, thereby reducing the ability of said third protein to bind to said first protein; and
   (v) testing said selected compounds in said mutated cell with a Wnt-dependent biological assay that measures a change in Wnt activity caused by the presence of said selected compounds.
4. A method for identifying of a molecule or compound that modulates the activity of a member of the Wnt signaling pathway comprising the steps of:
   (i) determining the structure of a first protein in said cell that is part of the Wnt signaling pathway;
   (ii) carrying out a mutational analysis of said first protein wherein said analysis defines a binding cavity on said first protein which interacts with a second protein involved in the Wnt pathway;
   (iii) carrying out a virtual screening of a library of compounds to assess the binding affinity of the compounds to said cavity;
   (iv) selecting compounds based on their binding affinity;
   (v) providing a cell with a mutated gene resulting in the reduction or elimination of the expression of a third protein involved in the Wnt signaling pathway; and
   (vi) testing said selected compounds in said mutated cell with a Wnt-dependent biological assay that measures a change in Wnt activity caused by the presence of said selected compounds.
5. A method for identifying a molecule or compound that modulates the activity of a member of the Wnt signaling pathway comprising the steps of:
   (i) determining the structure of a first protein that is part of the Wnt signaling pathway;
   (ii) carrying out a mutational analysis of said first protein wherein said analysis defines a binding cavity on said first protein which interacts with a second protein involved in the Wnt pathway;
   (iii) carrying out a virtual screening of a library of compounds to assess the binding affinity of the compounds to said cavity;
   (iv) selecting certain compounds based on their binding affinity;
   (v) providing a cell with a mutated gene resulting in the reduction or elimination of the expression of a third protein in the Wnt signaling pathway which binds with said first protein, thereby reducing the ability of said third protein to bind to said first protein; and
   (vi) testing said selected compounds in said mutated cell with a Wnt-dependent biological assay that measures a change in Wnt activity caused by the presence of said selected compounds.
6. The method of item 2, 3, 4 or 5 wherein said mutated gene comprises the elimination of all or a portion of said gene from a chromosome of said cell or animal.
7. The method of item 2, 3, 4 or 5 wherein said mutated gene comprises the insertion of at least one nucleic acid sequence into a chromosome of said cell or animal.
8. The method of item 7, wherein said insertion takes place within the sequence of said gene and thereby disrupts the expression or activity of said gene.
9. The method of item 2, 3, 4 or 5, wherein said mutated gene is a homozygous condition.
10. The method of item 2, 3, 4 or 5, wherein said mutated gene is a heterozygous condition.
11. The method of item 1, wherein said target protein comprises LRP5, LRP6, a member of the Wnt family, a member of the Dkk family, a member of the frizzled family, or a member of the disheveled (Dvl) family.
12. The method of item 2, 3, 4 or 5, wherein said mutated gene is directed towards an alteration in the expression of LRP5, LRP6, a member of the Wnt family, a member of the Dkk family, a member of the frizzled family, or a member of the disheveled family.
13. The method of item 2, 3, 4 or 5, wherein said mutated gene comprises the introduction of at least one nucleic acid sequence that directs expression of antisense or siRNA transcripts comprising sequences complementary to sequences in said gene.
14. The method of item 13, wherein said expression of antisense or siRNA is constitutive.
15. The method of item 13, wherein said expression of antisense or siRNA is tissue specific.
16. The method of item 13, wherein said expression of antisense or siRNA is inducible.
17. The method of item 1, 2, 3, 4 or 5 wherein said library of compounds comprises a physical library.
18. The method of item 1, wherein said library of compounds comprises a virtual library and said selected compounds are synthesized prior to carrying out step (v).
19. The method of item 1, wherein said molecule or compound modulates the interaction between two proteins wherein said first protein is said target protein and said second protein comprises a member of the Wnt signaling pathway.
20. The method of item 19, wherein said first protein and said second protein are a pair of proteins comprising Wnt and LRP5, Wnt and LRP6, Wnt and Dkk, Dkk and Kremen, frizzled and Wnt, or disheveled and frizzled.
21. The method of item 1, wherein said molecule or compound modulates the interaction between two proteins where said first protein is said target protein and said second protein does not comprise a member of the Wnt signaling pathway.
22. The method of item 21, wherein said first protein is LRP5 or LRP6 and said second protein comprises a protein of the anthrax LT complex.
23. The method of item 22, wherein said second protein is ANTRX1.
24. The method of item 22, wherein said second protein is ANTRX2.
25. The method of item 1, wherein said virtual screening has been carried out after carrying out at least one process comprising X-ray crystallography, site specific mutagenesis, NMR spectrography or homology modeling using a similar protein as a template for determination of the structure of said selected site.
26. A method for identifying a molecule or compound that modulates the activity of a member of the Wnt signaling pathway comprising the steps of:
   (i) carrying out a virtual screening of a library of compounds to select compounds which bind to a site on a target protein involved in Wnt signaling; and
   (ii) carrying out a biological assay of said selected compounds for modulation of Wnt activity in a cell or animal wherein said cell or animal comprises at least one genetically coded modification that affects the ability of a second protein involved in the Wnt signaling pathway to interact with a third protein.
27. The method of item 26, wherein said modification comprises the elimination or replacement of all or a portion of the sequence of the gene coding for said second protein.
28. The method of item 26, wherein said modification comprises a mutation that alters the identity of an amino acid of said second protein.
29. The method of item 26, wherein said modification comprises the insertion of at least one nucleic acid sequence into a chromosome of said cell or animal.
30. The method of item. 29, wherein said insertion takes place within the sequence of the gene coding for said second protein and thereby disrupts the expression or activity of said second protein.
31. The method of item 27, 28, 29 or 30, wherein said genetically coded modification is a homozygous condition.
32. The method of item 27, 28, 29 or 30, wherein said genetically coded modification is a heterozygous condition.
33. The method of item 26, wherein said target protein comprises LRP5, LRP6, a member of the Wnt family, a member of the Dkk family, a member of the frizzled family, or a member of the disheveled (Dvl) family.
34. The method of item 26, wherein said second protein comprises LRP5, LRP6, a member of the Wnt family, a member of the Dkk family, a member of the frizzled family, or a member of the disheveled (Dvl) family.
35. The method of item 26, wherein said molecule or compound modulates the interaction between said target protein and said third protein where said third protein comprises a member of the Wnt signaling pathway.
36. The method of item 35, wherein said target protein and said third protein comprise a pair of proteins comprising Wnt and LRP5, Wnt and LRP6, Wnt and Dkk, Dkk and Kremen, frizzled and Wnt, or disheveled and frizzled.
37. The method of item 26, wherein said molecules or compound modulates the interaction between said target protein and said third protein, wherein said third protein does not comprise a member of the Wnt signaling pathway.
38. The method of item 37, wherein said target protein is LRP5, said second protein is LRP6 and said third protein is a protein of the anthrax LT complex.
39. The method of item 37, wherein said target protein is LRP6, said second protein is LRP5 and said third protein is a protein of the anthrax LT complex.
40. The method of item 34 or 35, wherein said third protein is ANTRX1.
41. The method of item 34 or 35, wherein said third protein is ANTRX2.
42. The method of item 26, wherein said library of compounds comprises a physical library.
43. The method of item 26, wherein said library of compounds comprises a virtual library and said selected compounds are synthesized prior to carrying out step (ii).
44. The method of item 26, wherein said virtual screening has been carried out after carrying out at least one process comprising X-ray crystallography, site specific mutagenesis, NMR spectrography, or homology modeling using a similar protein as a template for determination of the structure of said selected site.
45. A method for identifying a molecule or compound that modulates the expression or activity of a target protein comprising LRP5, LRP6, a member of the Wnt family, a member of the Dkk family, a member of the frizzled family or a combination thereof, comprising the steps of:
   (i) carrying out a virtual screening of a library of compounds to select compounds which bind to a site on said target protein;
   (ii) providing cells or animals wherein said cells or animals comprise at least one genetically coded modification wherein:
      a) the expression of a gene for a second protein involved in the Wnt signaling pathway is reduced or eliminated by said modification;
      b) the ability of a second protein involved in the Wnt signaling pathway to interact with a third protein is affected by said modification; or
      c) a combination of both a) and b); and
   (iii) carrying out a biological assay of said selected compounds for modulation of Wnt activity in said cells or animals.
46. The method of item 45, wherein said modification comprises the elimination or replacement of all or a portion of the coding sequence of the gene coding for said second protein.
47. The method of item 45, wherein said modification comprises a mutation that alters the identity of an amino acid of said second protein.
48. The method of item 45, wherein said modification comprises the insertion of at least one nucleic acid sequence into a chromosome of said cell or animal.
49. The method of item 48, wherein said insertion takes place within the sequence of the gene coding for said second protein and thereby disrupts the expression or activity of said second protein.
50. The method of item 46, 47, 48 or 49, wherein said genetically coded modification is a homozygous condition.
51. The method of item 46, 47, 48 or 49, wherein said genetically coded modification is a heterozygous condition.
52. The method of item 45, wherein said genetically coded modification comprises the introduction of at least one nucleic acid sequence that directs expression of antisense or siRNA transcripts comprising sequences complementary to sequences of transcripts coding for said protein.
53. The method of item 52, wherein said expression of antisense or siRNA is constitutive.
54. The method of item 52, wherein said expression of antisense or siRNA is tissue specific.
55. The method of item 52, wherein said expression ofantisense or siRNA is inducible.
56. The method of item 45, wherein said library of compounds comprises a physical library.
57. The method of item 45, wherein said library of compounds comprises a virtual library and said selected compounds are synthesized prior to carrying out step (iii).
58. The method of item 45, wherein said second protein comprises LRP5, LRP6, a member of the Wnt family, a member of the Dkk family, a member of the frizzled family, or a member of the disheveled (Dvl) family.
59. The method of item 45, wherein said target protein and said second protein are members of the same gene family.
60. The method of item 59, wherein said target protein is LRP5 and said second protein is LRP6.
61. The method of item 59, wherein said target protein is LRP6 and said second protein is LRP5.
62. The method of item 59, wherein said target protein and said second protein are members of the Writ family.
63. The method of item 59, wherein said target protein and said second protein are members of the Dkk family.
64. The method of item 59, wherein said target protein and said second protein are members of the disheveled family.
65. The method of item 59, wherein said target protein and said second protein are members of the frizzled family.
66. The method of item 59, wherein said target protein and said third protein are the same protein.
67. The method of item 45, wherein said molecule or compound modulates the interaction between said target protein and said third protein where said third protein comprises a member of the Wnt signaling pathway.
68. The method of item 67, wherein said target protein and said third protein comprise a pair of proteins comprising Wnt and LRP5, Wnt and LRP6, Wnt and Dkk, Dkk and Kremen, frizzled and Wnt, or disheveled and frizzled.
69. The method of item 45, wherein said molecule or compound modulates the interaction between said target protein and said third protein, wherein said third protein does not comprise a member of the Wnt signaling pathway.
70. The method of item 69, wherein said target protein is LRP5 or LRP6 and said third protein comprises a protein of the anthrax LT complex.
71. The method of item 70, wherein said third protein is ANTRX1.
72. The method of item 70, wherein said third protein is ANTRX2.
73. The method of item 45, wherein said virtual screening has been carried out after carrying out at least one process comprising X-ray crystallography, site specific mutagenesis, NMR spectrography, or homology modeling using a similar protein as a template.
74. A method for identifying a molecule or compound that modulates the activity of a member of the Wnt signaling pathway comprising the steps of:
   (i) carrying out a virtual screening of a library of compounds to select compounds which bind to a site on a target protein involved in Wnt signaling;
   (ii) providing cells or animals wherein said cells or animals comprises at least one genetically coded modification wherein:
      a) the expression of a gene for a second protein involved in the Wnt signaling pathway is reduced or eliminated by said modification;
      b) the ability of a second protein involved in the Wnt signaling pathway to interact with a third protein is affected by said modification; or
      c) a combination of both a) and b) wherein said second protein comprises LRP5, LRP6, a member of the Wnt family, a member of the Dkk family or a member of the frizzled family; and
   (iii) carrying out a biological assay of selected compounds for modulation of Wnt activity in said cells or animals.
75. The method of item 74, wherein said modification comprises the elimination or replacement of all or a portion of the coding sequence of the gene coding for said second protein.
76. The method of item 74, wherein said modification comprises a mutation that alters the identity of an amino acid of said second protein.
77. The method of item 74, wherein said modification comprises the insertion of a nucleic acid sequence into a chromosome of said cell or animal.
78. The method of item 77, wherein said insertion takes place within the sequence of the gene coding for said second protein and thereby disrupts the expression or activity of said second protein.
79. The method of item 75, 76, 77 or 78, wherein said genetically coded modification is a homozygous condition.
80. The method of item 75, 76, 77 or 78, wherein said genetically coded modification is a heterozygous condition.
81. The method of item 74, wherein said genetically coded modification comprises the introduction of at least one nucleic acid sequence that directs expression of antisense or siRNA transcripts comprising sequences complementary to sequences of transcripts coding for said protein.
82. The method of item 81, wherein said expression of antisense or siRNA is constitutive.
83. The method of item 81, wherein said expression of antisense or siRNA is tissue specific.
84. The method of item 81, wherein said expression of antisense or siRNA is inducible.
85. The method of item 74, wherein said library of compounds comprises a physical library.
86. The method of item 74, wherein said library of compounds comprises a virtual library and said selected compounds are synthesized prior to carrying out step (iii).
87. The method of item 74, wherein said target protein comprises LRP6, LRP6, a member of the Wnt family, a member of the Dkk family, a member of the frizzled family, or a member of the disheveled (Dvl) family.
88. The method of item 74, wherein said target protein and said second protein are members of the same gene family.
89. The method of item 88, wherein said target protein is LRP5 and said second protein is LRP6.
90. The method of item 88, wherein said target protein is LRP6 and said second protein is LRP5.
91. The method of item 74, wherein said target protein and said third protein are the same protein.
92. The method of item 74, wherein said molecule or compound modulates the interaction between said target protein and said third protein, wherein said third protein comprises a member of the Wnt signaling pathway.
93. The method of item 92, wherein said target protein and said third protein comprise a pair of proteins comprising Wnt and LRP5, Wnt and LRP6, Wnt and Dkk, Dkk and Kremen, frizzled and Wnt, or disheveled and frizzled.
94. The method of item 74, wherein said molecule or compound modulates the interaction between said target protein and said third protein, wherein said third protein does not comprise a member of the Wnt signaling pathway.
95. The method of item 94, wherein said target protein is LRP5 or LRP6 and said third protein comprises a protein of the anthrax LT complex.
96. The method of item 95, wherein said third protein is ANTRX1.
97. The method of item 95, wherein said third protein is ANTRX2.
98. The method of item 74, wherein said second protein and said third protein comprise a pair of proteins comprising Wnt and LRP5, Wnt and LRP6, Wnt and Dkk, Dkk and Kremen, frizzled and Wnt, or disheveled and frizzled.
99. A method for identifying a molecule or compound that modulates the activity of a member of the Wnt signaling pathway comprising the steps of:
   (i) carrying out a virtual screening of a library of compounds to select compounds which bind to a site on a target protein involved in Went signaling; and
   (ii) carrying out a biological assay of said selected compounds for modulation of Wnt activity in a cell or animal wherein said cell or animal comprises at least one genetically coded modification wherein the expression or activity of a gene involved in the Wnt signaling pathway is reduced or eliminated by:
      a) elimination of all or a portion of said gene from a chromosome of said cell or animal;
      b) insertion of a nucleic acid into a chromosome of said cell or animal wherein said insertion takes place within the sequence of said gene; or
      c) introduction of one or more nucleic acid sequences that directs expression of antisense RNA or siRNA transcripts that are complementary to sequences in said gene.
100. The method of item 99, wherein said genetically coded modification is a homozygous condition.
101. The method of item 99, wherein said genetically coded modification is a heterozygous condition.
102. The method of item 99, wherein said genetically coded modification is directed towards an alteration in the expression or activity of LRP5, LRP6, a member of the Wnt family, a member of the Dkk family, a member of the frizzled family, or a member of the disheveled family.
103. The method of item 99, wherein said expression of antisense or siRNA is constitutive.
104. The method of item 99, wherein said expression of antisense or siRNA is tissue specific.
105. The method of item 99, wherein said expression of antisense or siRNA is inducible.
106. A method for identifying a molecule or compound that modulates the expression or activity of a target protein comprising LRP5, LRP6, a member of the Wnt family, a member of the Dkk family, a member of the Frizzled family, or a combination thereof, comprising the steps of:
   (i) carrying out a virtual screening of a library of compounds to select compounds which bind to a site on said target protein;
   (ii) providing cells or animals wherein said cells or animals comprise at least one genetically coded modification wherein:
      a) the expression of a gene for a second protein involved in the Wnt signaling pathway is reduced or eliminated by said modification;
      b) the ability of a second protein involved in the Wnt signaling pathway to interact with a third protein is affected by said modification; or
      c) a combination of both a) and b) wherein the expression or activity of said gene involved in the Wnt signaling pathway is reduced or eliminated by:
         1) elimination of all or a portion of said gene from a chromosome of said cell or animal;
         2) insertion of a nucleic acid into a chromosome of said cell or animal wherein said insertion takes place within the sequence of said gene; or
         3) introduction of one or more nucleic acid sequences that directs expression of antisense RNA or siena transcripts that are complementary to sequences in said gene; and
   (iii) carrying out a biological assay of said selected compounds for modulation of Wnt activity in said cells or animals.
107. The method of item 106, wherein said genetically coded modification is a homozygous condition.
108. The method of item 106, wherein said genetically coded modification is a heterozygous condition.
109. The method of item 106, wherein said expression of antisense or siRNA is constitutive.
110. The method of item 106, wherein said expression of antisense or siRNA is tissue specific.
111. The method of item 106, wherein said expression of antisense or siRNA is inducible.
112. The method of item 106, wherein said second protein comprises LRP5, LRP6, a member of the Wnt family, a member of the Dkk family, a member of the frizzled family, or a member of the disheveled (Dvl) family.
113. The method of item 106, wherein said target protein and said second protein are members of the same gene family.
114. A method for identifying a molecule or compound that modulates the activity of a member of the Wnt signaling pathway comprising the steps of:
   (i) carrying out a virtual screening of a library of compounds to select compounds which bind to a site on a target protein involved in Wnt signaling;
   (ii) providing cells or animals wherein said cells or animals comprise at least one genetically coded modification wherein:
      a) the expression of a gene for a second protein involved in the Wnt signaling pathway is reduced or eliminated by said modification
      b) the ability of a second protein involved in the Wnt signaling pathway to interact with a third protein is affected by said modification; or
      c) a combination of both a) and b) wherein the expression or activity of said gene involved in the Wnt signaling pathway is reduced or eliminated by:
         1) elimination of all or a portion of said gene from a chromosome of said cell or animal;
         2) insertion of a nucleic acid into a chromosome of said cell or animal wherein said insertion takes place within the sequence of said gene; or
         3) introduction of one or more nucleic acid sequences that directs expression of antisense RNA or siRNA transcripts that are complementary to sequences in said gene and wherein said second protein comprises LRP5, LRP6, a member of the Wnt family, a member of the Dkk family or a member of the frizzled family; and
   (iii) carrying out a biological assay of said selected compounds from step (i) for modulation of Wnt activity in said cells or animals.
115. The method of item 114, wherein said genetically coded modification is a homozygous condition.
116. The method of item 114, wherein said genetically coded modification is a heterozygous condition.
117. The method of item 114, wherein said expression of antisense or siRNA is constitutive.
118. The method of item 114, wherein said expression of antisense or siRNA is tissue specific.
119. The item 114, wherein said expression of antisense or siRNA is inducible.
120. The method of item 114, wherein said third protein comprises LRP5, LRP6, a member of the Wnt family, a member of the Dkk family, a member of the frizzled family, or a member of the disheveled (Dvl) family.
121. The method of item 114, wherein said target protein and said second protein are members of the same gene family.
122. The method of item 114, wherein said target protein and said third protein are the same protein.
123. The method of item 1, 2, 3, 4, 5, 26, 45, 74, 99, 106 or 114, wherein said activity comprises participation in anthrax toxicity.
124. A method for treating a patient comprising administration of a molecule or compound that has been selected by the methods of any one of items 1 to 123.
125. A method for identifying a molecule or compound useful for decreasing the toxic effects of anthrax exposure comprising the steps of:
   (i) carrying out a virtual screening of a library of compounds to select compounds which bind to a selected site on LRP6; and
   (ii) carrying out a biological assay of said selected compounds for toxicity towards a cell or animal after exposure to a complex comprising PA + EF, PA + LF, or PA + EF + LF.
126. The method of item 125, wherein said selected site is Domain II of LRP6, Domain III of LRP6, or the extracellular portion of LRP6 that is adjacent to the trans membrane domain of LRP6.
127. The method of item 125, comprising a further step of mutational scanning for identifying ammo acids important in interactions between LRP6 and at least one protein comprising the anthrax toxin complex.
128. The method of item 125, comprising a further step of carrying out a structural determination to identify the interaction sites between LRP6 and at least one protein comprising the anthrax toxin complex.
129. The method of item 127 or 128, wherein said protein comprises ANTRX1.
130. The method of item 127 or 128, wherein said protein comprises ANTRX2.
131. The method of item 125, wherein said library of compounds comprises a physical library.
132. The method of item 125, wherein said library of compounds comprises a virtual library and said selected compounds are synthesized prior to carrying out step (ii).
133. The method of item 125, wherein prior to carrying out said biological assay, a first biological assay is carried out based upon the ability of a selected compound to modulate Wnt activity.
134. The method of item 125, wherein said biological assay is carried out in cells or animals wherein said cells or animals comprise at least one genetically coded modification wherein:
   a) the expression of a gene for a protein involved in the Wnt signaling pathway is reduced or eliminated by said modification;
   b) the ability of a protein involved in the Wnt signaling pathway to interact with a third protein is affected by said modification; or
   c) a combination of both a) and b).
135. A method for identifying a molecule or compound useful for decreasing the toxic effects of anthrax exposure comprising the steps of:
   (i) carrying out a virtual screening of a library of compounds to select compounds which bind to a selected site on LRP5; and
   (ii) carrying out a biological assay of compounds selected from step (i) for toxicity towards a cell or animal after exposure to a complex comprising PA + EF, PA + LF or PA + EF + LF.
136. The method of item 135, wherein said selected site is Domain II of LRP5, Domain III of LRP5, or the extracellular portion of LRP5 that is adjacent to the transmembrane domain of LRP5.
137. The method of item 135, comprising a further step of mutational scanning for identifying amino acids important in interactions between LRP5 and at least one protein comprising the anthrax toxin complex.
138. The method of item 135, comprising a further step of carrying out a structural determination to identify the interaction sites between LRP5 and at least one protein comprising the anthrax toxin complex.
139. The method of item 137 or 138, wherein said protein comprises ANTRX1.
140. The method of item 137 or 138, wherein said protein comprises ANTRX2.
141. The method of item 135, wherein said library of compounds comprises a physical library.
142. The method of item 135, wherein said library of compounds comprises a virtual library and said selected compounds are synthesized prior to carrying out step (ii).
143. The method of item 135, wherein prior to carrying out said biological assay, a first biological assay is carried out based upon the ability of a selected compound to modulate Wnt activity.
144. The method of item 135, wherein said biological assay is carried out in cells or animals wherein said cells or animals comprise at least one genetically coded modification wherein:
   a) the expression of a gene for a protein involved in the Wnt signaling pathway is reduced or eliminated by said modification;
   b) the ability of a involved in the Wnt signaling pathway to interact with a third protein is affected by said modification; or
   c) a combination of both a) and b).
145. A method of treating anthrax toxicity by administering an organic compound that has been selected by a virtual screening of a library of compounds for binding to LRP6.
146. The method of item 145, wherein said library of compounds represents a physical library.
147. The method of item 145, wherein said selection further comprises a biological assay.
148. The method of item 147, wherein said biological assay comprises one or more assays selected from:
   a) binding between LRP6 and another protein;
   b) modulation of Wnt activity;
   c) effects upon cell death by anthrax toxin; and
   d) a combination thereof.
149. The method of item 145, wherein said library of compounds represents a virtual library.
150. The method of item 145, wherein said selection further comprises:
   (i) synthesis of one or more compounds after said synthesized virtual screening; and
   (ii) a biological assay with said synthesized compounds.
151. The method of item 150, wherein said biological assay comprises one or more assays selected from:
   a) binding between LRP6 and another protein;
   b) modulation of Wnt activity; and
   c) effects upon cell death by anthrax toxin.
152. A method of treating anthrax toxicity by administering an organic compound that has been selected by a virtual screening of a library of compounds for binding to LRP5.
153. The method of item 152, wherein said library of compounds represents a physical library.
154. The method of item 152, therein said selection further comprises a biological assay.
155. The method of item 154, wherein said biological assay comprises:
   a) binding between LRP5 and another protein;
   b) modulation of Wnt activity;
   c) effects upon cell death by anthrax toxin; and
   d) a combination thereof.
156. The method of item 152, wherein said library of compounds represents a virtual library.
157. The method of item 152, wherein said selection further comprises:
   (i) synthesis of one or more compounds after said virtual screening; and
   (ii) a biological assay with said synthesized compounds.
158. The method of item 157, wherein said biological assays comprises one or more assays selected from binding between LRP6 and another protein, modulation of Wnt activity and effects upon cell death by anthrax toxin.
159. A method for treating a patient comprising administration of a molecule or compound that has been selected by the methods of any one of items 125 to 158.
160. A composition comprising the molecule or compound identified in any one of items 1 to 158.

## Claims

1. A method for identifying a molecule or compound useful for decreasing the toxic effects of anthrax exposure comprising the steps of:
(i) carrying out a virtual screening of a library of compounds to select compounds which bind to a selected site on LRP5 or LRP6; and
(ii) carrying out a biological assay of said selected compounds for toxicity towards a cell or animal after exposure to a complex comprising PA + EF, PA + LF, or PA + EF + LF,
wherein PA is anthrax Protective Antigen, EF is anthrax Edema Factor, and LF is anthrax Lethal Factor.

2. The method of claim 1, wherein said selected site is Domain II of LRP5 or LRP6, Domain III of LRP5 or LRP6, or the extracellular portion of LRP5 or LRP6 that is adjacent to the trans membrane domain of LRP5 or LRP6, respectively.

3. The method of claim 1, comprising a further step of mutational scanning for identifying amino acids important in interactions between LRP5 or LRP6 and at least one protein comprising the anthrax toxin complex.

4. The method of claim 1, comprising a further step of carrying out a structural determination to identify the interaction sites between LRP5 or LRP6 and at least one protein comprising the anthrax toxin complex.

5. The method of claim 3 or 4, wherein said protein comprises ANTRX1.

6. The method of claim 3 or 4, wherein said protein comprises ANTRX2.

7. The method of claim 1, wherein said library of compounds comprises a physical library.

8. The method of claim 1, wherein said library of compounds comprises a virtual library and said selected compounds are synthesized prior to carrying out step (ii).

9. The method of claim 1, wherein prior to carrying out said biological assay, a first biological assay is carried out based upon the ability of a selected compound to modulate Wnt activity.

10. The method of claim 1, wherein said biological assay is carried out in cells or animals wherein said cells or animals comprise at least one genetically coded modification wherein
a) the expression of a gene for a protein involved in the Wnt signaling pathway is reduced or eliminated by said modification; or
b) the ability of a protein involved in the Wnt signaling pathway to interact with a third protein is affected by said modification; or
c) a combination of both a) and b).
